# EUROPÄISCHE PATENTSCHRIFT

(11) **EP 4 205 711 B1**
(45) Veröffentlichungstag und Bekanntmachung des Hinweises auf die Patenterteilung: **28.08.2024**
(21) Anmeldenummer: 21218176.2
(22) Anmeldetag: 29.12.2021
(51) Int. Cl.: A61F 13/02

(54) **SELBSTKLEBENDE ATMUNGSAKTIVE WUNDAUFLAGE FÜR STARK EXSUDIERENDE WUNDEN**
SELF-ADHESIVE BREATHABLE WOUND DRESSING FOR HIGHLY EXUDING WOUNDS
PANSEMENT RESPIRANT AUTO-ADHÉSIF POUR PLAIES À EXSUDAT FORT

(43) Veröffentlichungstag der Anmeldung: 05.07.2023
(73) Patentinhaber: Paul Hartmann AG, 89522 Heidenheim (DE)
(72) Erfinder: Streit, Iris, 89518 Heidenheim (DE)
(74) Vertreter: Paul Hartmann AG Patents & Licensing

(56) Entgegenhaltungen:
- DE-A1- 102006 031 418
- US-A1- 2017 128 270
- US-A1- 2019 192 352
- US-A1- 2019 358 094
- US-A1- 2019 365 580

## Beschreibung

Die vorliegende Erfindung betrifft eine Wundauflage umfassend eine Rückschicht, ein Wundkissen und eine adhäsive Schicht. Zudem betrifft die Erfindung ein Verfahren zur Herstellung der Wundauflage.

Stark exsudierende Wunden werden heutzutage bevorzugt mit superabsorbierenden Wundauflagen behandelt. Diese Wundauflagen werden entweder mit Hilfe von Sekundärverbanden am Körper des Patienten befestigt oder sie sind selbstklebend ausgestaltet. Der Vorteil der selbstklebenden Ausgestaltung liegt darin, dass der Anwender die Wundauflage einfacher und schneller anlegen kann und nicht etwa auf mehrere, separat bereitgestellte Verbandsmaterialien zurückgreifen muss, um die Wunde zu versorgen.

Oft weisen selbstklebende absorbierende Wundauflagen eine adhäsiv beschichtete Rückschicht aus einer Polyurethanfolie auf. Diese kann vorteilhafterweise keim- und wasserdicht sein, gleichzeitig jedoch die Atmungsaktivität beziehungsweise die Wasserdampfdurchlässigkeit der Wundauflage einschränken. Die eingeschränkte Atmungsaktivität kann dazu führen, dass die Wundauflage schneller mit Wundexsudat gesättigt ist und sich die Gefahr einer Mazeration der Wunde erhöht. Zudem empfinden manche Anwender Wundauflagen mit einer Rückschicht aus einer Polyurethanfolie als eher künstlich und weniger angenehm.

Einige im Markt verfügbare selbstklebende absorbierende Wundauflagen verfügen über eine Silikonwundkontaktschicht in der Art einer perforierten, adhäsiv beschichteten Folie, welche die komplette Unterseite der Wundauflage bildet und in ihrem Randbereich mit der Rückschicht der Wundauflage verbunden ist. Dabei sind die absorbierenden Bestandteile der Wundauflage sandwichartig zwischen der Rückschicht und der Silikonwundkontaktschicht eingeschlossen. Mit der Silikonwundkontaktschicht wird die Wundauflage am Körper des Patienten befestigt. Da der verwendete Silikonkleber nur vergleichsweise schwach an der Haut und der Wundoberfläche haftet, kann ein schonender Verbandswechsel erfolgen.

Grundsätzlich wirkt die zuvor genannte Silikonwundkontaktschicht aufgrund ihrer hydrophoben Ausgestaltung als Barriere für die Aufnahme von Wundexsudat. Dieses Problem wird damit gelöst, indem die Wundkontaktschicht mit Perforationen in geeigneter Anzahl und Größe für den Durchtritt der Wundflüssigkeit versehen ist. Die Anzahl und Größe der Perforationen können dabei aber üblicherweise nicht beliebig hoch gewählt werden. Denn ansonsten könnte zum Beispiel die zuverlässige Haftung der Wundauflage an der Wundstelle nicht mehr gegeben sein. Jedenfalls kann die zuvor genannte Silikonwundkontaktschicht aufgrund ihrer begrenzten Durchlässigkeit für Wundexsudat auch dazu führen, dass das Wundexsudat gegebenenfalls nur noch vergleichsweise langsam von der Wundauflage absorbiert werden kann. Dieser Nachteil kommt insbesondere dann zum Tragen, wenn das Wundexsudat viskos ist.

Die US 2017/0128270 A1 offenbart einen Inselverband, bei dem ein SAP-haltiges absorbierendes Material mittels einem Verankerungsmaterial an einer adhäsiven Rückschicht befestigt ist.

Die DE 10 2006 031 418 A1 offenbart einen Absorptionskörper zur Auflage an menschliche sowie tierische Wundflächen im Rahmen eines Okklusiv- oder Semiokklusivverbands umfassend eine SAP-haltige, flüssigkeitsabsorbierende Matte, die von einer wenigstens teilweise perforierten Hülle umgeben ist.

Die Aufgabe der vorliegenden Erfindung bestand darin, eine selbstklebende, absorbierende Wundauflage zur Behandlung von Wunden, insbesondere zur Behandlung von stark exsudierenden Wunden, bereitzustellen, welche die zuvor beschriebenen Nachteile nicht aufweist. Entsprechend sollte eine selbstklebende, absorbierende Wundauflage gefunden werden, welche eine hohe Atmungsaktivität aufweisen kann und Wundexsudat schnell und in großer Menge aufnehmen kann. Zudem sollte sich die Wundauflage angenehm und weich anfühlen sowie einfach und kostengünstig herstellbar sein. Die Erfindung löst diese Aufgaben mit einer Wundauflage nach Anspruch 1 und einem Verfahren zur Herstellung einer Wundauflage nach Anspruch 15.

Die erfindungsgemäße Wundauflage zeichnet sich durch die nachfolgenden Merkmale aus. Die Wundauflage umfasst eine Rückschicht bestehend aus einem Vliesstoff. Dadurch kann die Wundauflage sehr atmungsaktiv beziehungsweise wasserdampfdurchlässig sein, um insbesondere einer Mazeration der Wunde vorzubeugen. Durch den für die Rückschicht verwendeten Vliesstoff kann sich die Wundauflage auch angenehm und weich anfühlen.

Weiterhin umfasst die Wundauflage ein Wundkissen umfassend eine Hülle und einen absorbierenden Kern mit superabsorbierenden Polymer-Partikeln (SAP-Partikeln). Der absorbierende Kern befindet sich in der Hülle und wird von dieser umschlossen. Das Wundkissen stellt die absorbierende Komponente der Wundauflage dar. Indem das Wundkissen SAP-Partikel enthält, kann die Wundauflage sehr große Mengen an Wundflüssigkeit aufnehmen und binden. Zudem umfasst die Wundauflage eine erste adhäsive Schicht. Diese ist zwischen der Rückschicht und dem Wundkissen angeordnet, wobei die Rückschicht und die erste adhäsive Schicht das Wundkissen überfangen, so dass die Wundauflage einen adhäsiven Rand aufweist und selbstklebend ausgestaltet ist. Mit dem adhäsiven Rand kann die Wundauflage einfach und schnell an einer Wundstelle befestigt werden. Von besonderer Bedeutung ist ferner, dass die Wundauflage keine mit dem adhäsiven Rand und einer wundzugewandten Seite der Hülle des Wundkissens unlösbar verbundene Schicht umfasst. Dadurch kann die Wundauflage wie bereits beschrieben mit dem adhäsiven Rand an der Wundstelle befestigt werden. Ein weiterer vorteilhafter Effekt davon ist, dass die wundzugewandte Seite der Hülle des Wundkissens eine Wundkontaktschicht der Wundauflage bilden kann. Das Wundkissen kann dann mit seiner in der Anwendung der Wundauflage wundzugewandten Seite vollflächig mit der Wundoberfläche in Kontakt treten und für die Aufnahme von Wundexsudat zur Verfügung stehen. Somit kann die Wundauflage die Wundflüssigkeit schnell und ungehindert aufnehmen.

Die erfindungsgemäße Wundauflage ist also in der Art eines Inselverbands ausgestaltet. Ein Sandwich-Design, wonach das Wundkissen wundseitig von einer zusätzlichen, nicht ablösbaren Verbandslage dauerhaft bedeckt wäre, wurde aus den zuvor genannten Gründen nicht gewählt.

Mit der erfindungsgemäßen Wundauflage können auch mittel bis stark exsudierende, akute sowie chronische Wunden effektiv und schonend behandelt werden. Weitere Vorteile der Wundauflage ergeben sich aus den nachfolgend beschriebenen bevorzugten Ausführungsformen.

Der Vliesstoff der Rückschicht ist vorzugsweise ein wasserstrahlverfestigter Vliesstoff (Spunlaced-Vliesstoff). Im Hinblick auf das Fasermaterial für den Vliesstoff der Rückschicht haben sich Polyesterfasern als besonders geeignet herausgestellt. Entsprechend besteht der Vliesstoff der Rückschicht in einer bevorzugten Ausführungsform der Erfindung aus Polyesterfasern.

In einer bevorzugten Ausführungsform der Erfindung ist die Rückschicht perforiert, um die Wasserdampfdurchlässigkeit der Rückschicht weiter zu verbessern. Dabei sind vorzugsweise eine Vielzahl von Perforationen im Wesentlichen gleichmäßig über die Rückschicht verteilt vorhanden. Die Perforationen können eine Größe von 0,1 mm bis 2 mm, bevorzugt von 0,1 mm bis 1 mm und insbesondere von 0,25 mm bis 1 mm, aufweisen und im Wesentlichen rechteckig, oval oder rund ausgestaltet sein, wobei sich die Größenangabe dann auf die Länge des Rechtecks beziehungsweise Ovals oder auf den Kreisdurchmesser bezieht.

Die hohe Atmungsaktivität der Wundauflage kann auch messtechnisch erfasst und angegeben werden. So kann die Rückschicht, vorzugsweise die Rückschicht in Verbindung mit der ersten adhäsiven Schicht und insbesondere die Wundauflage in ihrer Gesamtheit, eine Wasserdampfdurchlässigkeit (MVTR - moisture vapor transmission rate) von mindestens 3000 g/m²/24h, vorzugsweise von mindestens 3500 g/m²/24h und insbesondere von mindestens 4000 g/m²/24h, gemessen nach DIN EN 13726-2:2002-06, aufweisen. Zudem kann die Rückschicht, vorzugsweise die Rückschicht in Verbindung mit der ersten adhäsiven Schicht und insbesondere die Wundauflage in ihrer Gesamtheit, eine Wasserdampfdurchlässigkeit (MVTR) von maximal 5000 g/m²/24h, gemessen nach DIN EN 13726-2:2002-06, aufweisen. Bevorzugt weist die Rückschicht, vorzugsweise die Rückschicht in Verbindung mit der ersten adhäsiven Schicht und insbesondere die Wundauflage in ihrer Gesamtheit, eine Wasserdampfdurchlässigkeit (MVTR) von 3000 g/m²/24h bis 5000 g/m²/24h, gemessen nach DIN EN 13726-2:2002-06, auf.

Für die Bestimmung der Wasserdampfdurchlässigkeit gemäß DIN EN 13726-2:2002-06 ist vorliegend folgendes zu beachten:
1. Die Bestimmung erfolgt gemäß Kapitel 3.2 (MVTR einer Wundauflage bei Kontakt mit Wasserdampf).
2. Wenn die Bestimmung an der gesamten Wundauflage erfolgen soll, werden die Proben aus einem zentralen Bereich der Wundauflage entnommen und nicht aus dem adhäsiven Randbereich der Wundauflage, damit die Proben alle Schichten der Wundauflage umfassen.
3. Die nachfolgend beschriebene Abziehfolie wird insofern vorhanden von der Wundauflage entfernt, bevor die Wasserdampfdurchlässigkeit bestimmt wird.

Typischerweise sind die Rückschicht und die erste adhäsive Schicht gleich groß ausgebildet und das Wundkissen ist kleiner als die Rückschicht und die erste adhäsive Schicht ausgebildet, wobei das Wundkissen vorzugsweise im Wesentlichen mittig auf der ersten adhäsiven Schicht angeordnet ist. Es ergibt sich dann die bereits erwähnte "Insel"-Anordnung der Wundauflage, welche zur Lösung der vorliegenden Aufgabenstellung besonders geeignet ist.

In einer bevorzugten Ausgestaltung der Erfindung ist die erste adhäsive Schicht als Beschichtung einer wundzugewandten Seite der Rückschicht ausgebildet. Diese Ausgestaltung ist einfach umsetzbar und kostengünstig.

Weiterhin kann die erste adhäsive Schicht eine wundzugewandte Seite der Rückschicht vollflächig beziehungsweise vollständig bedecken. Dann ist eine besonders hohe Haftung der Wundauflage an der Wundstelle möglich. Alternativ kann die erste adhäsive Schicht eine wundzugewandte Seite der Rückschicht nur teilweise bedecken. Mit dieser Maßnahme kann die Wasserdampfdurchlässigkeit der Wundauflage weiter verbessert werden, weshalb diese Ausführungsform vorliegend bevorzugt ist. Die erste adhäsive Schicht kann dabei zum Beispiel perforiert sein oder insbesondere voneinander beabstandete Streifen ausbilden, wobei die erste adhäsive Schicht dann vorzugsweise ein gelochtes oder gestreiftes Muster ausbildet, welches auf der gesamten wundzugewandten Seite der Rückschicht vorhanden ist. Die adhäsiven Streifen können eine Breite von 0,5 mm bis 2 mm aufweisen. Ebenso kann der Abstand der adhäsiven Streifen untereinander 0,5 mm bis 2 mm betragen.

Die erste adhäsive Schicht kann einen Acrylatkleber, einen synthetischen Kautschuk-Kleber oder einen Silikonkleber umfassen. Vorzugsweise besteht die erste adhäsive Schicht aus einem Acrylatkleber, einem synthetischen Kautschuk-Kleber oder einem Silikonkleber. Acrylatkleber und Kautschuk-Kleber können eine hohe Haftkraft aufweisen und kostengünstig sein. Silikonkleber kann gewebeschonend (atraumatisch) sein, ist in der Regel aber teurer als die beiden anderen zuvor genannten Klebstoffvarianten. Bevorzugt wird für die vorliegende Erfindung der synthetische Kautschuk-Kleber verwendet. Besonders bevorzugt ist vorliegend eine gestreifte Beschichtung der Rückschicht mit synthetischem Kautschuk-Kleber. Dies ist vorteilhaft im Hinblick auf die Haftkraft, die Wasserdampfdurchlässigkeit sowie die Produktionskosten der Wundauflage.

Vorliegend ist es mit Blick auf eine einfache und kostengünstige Ausgestaltung der Wundauflage insbesondere vorgesehen, dass die Wundauflage aus der Rückschicht, dem Wundkissen, der ersten adhäsiven Schicht und optional einer einteiligen oder mehrteiligen, insbesondere zweiteiligen, Abziehfolie besteht. Die Abziehfolie bedeckt den adhäsiven Rand und die wundzugewandte Seite der Hülle des Wundkissens und kann von der Wundauflage abgezogen werden. Die Abziehfolie schützt die für den Haut- und Wundkontakt vorgesehene Unterseite der Wundauflage vor Kontamination während der Lagerung und verbessert die Handhabbarkeit der Wundauflage. Abziehfolien als Applikationshilfe für Wundauflagen sind an sich bekannt und im Stand der Technik vielfach beschrieben. Die Abziehfolie kann zum einfacheren Ablösen von der Wundauflage einseitig silikonisiert sein und zum Beispiel Pergaminpapier, Polyethylen oder Polypropylen umfassen. Bevorzugt handelt es sich bei der Abziehfolie um Silikon-Pergaminpapier.

Abweichend von der zuletzt beschriebenen Ausführungsform kann es jedoch auch vorgesehen sein, dass die Wundauflage neben der Rückschicht, dem Wundkissen, der ersten adhäsiven Schicht und gegebenenfalls der Abziehfolie noch weitere Schichten umfasst. So kann beispielsweise zwischen der Rückschicht und dem Wundkissen eine perforierte Folie, insbesondere eine perforierte Polyurethanfolie, angeordnet sein. Eine wundabgewandte Seite der Folie, welche das Wundkissen überfängt, ist mit einem Acrylatkleber beschichtet. Eine wundzugewandte Seite der Folie ist mit einem Silikonkleber beschichtet. Der Acrylatkleber bildet eine zweite adhäsive Schicht der Wundauflage und dient zur Verbindung der Folie mit der Rückschicht. Der Silikonkleber bildet die erste adhäsive Schicht der Wundauflage. Die erste adhäsive Schicht der Wundauflage dient zur Verbindung der Folie mit dem Wundkissen und zur Befestigung der Wundauflage an der Wundstelle. Die Beschichtung aus dem Silikonkleber stellt somit die erste adhäsive Schicht gemäß Anspruch 1 dar. Die Beschichtung aus dem Acrylatkleber stellt dann eine weitere, zweite adhäsive Schicht dar, die die Wundauflage in dieser Ausführungsform beinhaltet. Technisch vorteilhaft bei dieser Ausführungsform ist hauptsächlich, dass ein Eindringen der Klebstoffe in das Vliesstoffmaterial der Rückschicht unterbunden werden kann.

Anknüpfend an die zuletzt genannte Ausführungsform der Erfindung kann die Wundauflage dann aus der Rückschicht, dem Wundkissen, der beidseitig adhäsiv beschichteten Folie wie zuvor beschrieben und optional einer einteiligen oder mehrteiligen, insbesondere zweiteiligen, Abziehfolie bestehen. Die Abziehfolie bedeckt den adhäsiven Rand und die wundzugewandte Seite der Hülle des Wundkissens und kann von der Wundauflage abgezogen werden. Der Vorteil und spezielle Ausführungsformen der Abziehfolie sind bereits beschrieben worden.

Wie bereits dargestellt umfasst die erfindungsgemäße Wundauflage als wesentliche Komponente ein Wundkissen. Dessen bevorzugte Ausgestaltung soll nun im Folgenden genauer erläutert werden.

Gemäß einer besonders bevorzugten Ausführungsform der Erfindung besteht die Hülle des Wundkissens aus einer ersten, wundabgewandten hydrophoben Vliesstoffschicht und einer zweiten, wundzugewandten hydrophilen und dadurch absorbierenden Vliesstoffschicht. Dabei bildet die zweite, wundzugewandte hydrophile Vliesstoffschicht die Wundkontaktschicht der Wundauflage. Die erste Vliesstoffschicht ist hydrophob, weist also wasserabweisende Eigenschaften auf. Die zweite Vliesstoffschicht ist hydrophil, weist also wasseranziehende und dadurch absorbierende Eigenschaften auf. Wie nachfolgend noch deutlich wird, ist damit jedoch nicht ausgeschlossen, dass die zweite Vliesstoffschicht neben hydrophilen Komponenten auch hydrophobe Komponenten aufweist.

Normalerweise sind sowohl die erste als auch die zweite Vliesstoffschicht der Hülle kontinuierlich, also ohne die Schichten vollständig durchdringende Einschnitte, Perforationen oder sonstige Öffnungen, ausgebildet, damit insbesondere die SAP-Partikel nicht aus dem Wundkissen austreten können. Typischerweise sind die erste und zweite Vliesstoffschicht der Hülle ebenso wie der Vliesstoff der Rückschicht wasserdampfdurchlässig.

Zudem ist die erste Vliesstoffschicht der Hülle vorteilhafterweise im Wesentlichen flüssigkeitsundurchlässig ausgebildet. Hingegen muss die zweite Vliesstoffschicht der Hülle flüssigkeitsdurchlässig ausgebildet sein. Weiterhin ist die zweite Vliesstoffschicht der Hülle vorzugsweise nicht-verklebend ausgebildet, so dass ein schmerzfreier Verbandswechsel möglich ist.

Die erste Vliesstoffschicht kann aufgrund ihrer hydrophoben Eigenschaften im Wesentlichen flüssigkeitsundurchlässig sein und somit verhindern, dass die von dem Wundkissen aufgenommene Wundflüssigkeit mit der ersten adhäsiven Schicht sowie der Rückschicht in Kontakt kommen kann. Diese beiden, das Wundkissen bedeckenden Schichten können dann während der Anwendung der Wundauflage trocken bleiben und werden nicht mit Wundexsudat kontaminiert. Die zweite Vliesstoffschicht kann aufgrund ihrer hydrophilen Eigenschaften auch ohne Einschnitte, Perforationen oder sonstige in den Vliesstoff eingebrachte Öffnungen im hohen Maße flüssigkeitsdurchlässig sein und somit einen schnellen Flüssigkeitseintritt in das Wundkissen hinein gewährleisten.

Die erste Vliesstoffschicht der Hülle kann zum Beispiel aus Polypropylenfasern bestehen. Die zweite Vliesstoffschicht der Hülle kann aus Polyamidfasern und Viskosefasern bestehen. Dabei können 50 Gew.-% bis 60 Gew.-%, insbesondere etwa 55 Gew.-%, Polyamidfasern und 40 Gew.-% bis 50 Gew.-%, insbesondere etwa 45 Gew.-%, Viskosefasern bezogen auf das Gesamtgewicht der zweiten Vliesstoffschicht vorgesehen sein.

Weiterhin kann die als Wundkontaktschicht vorgesehene zweite Vliesstoffschicht der Hülle vorteilhafterweise zweischichtig aufgebaut sein. Die beiden Schichten können wie zuvor angegeben aus hydrophoben Polyamidfasern und hydrophilen Viskosefasern bestehen, wobei die eine Außenseite der Hülle bildende Schicht zur Verringerung der Verklebungsneigung mit der Wundoberfläche einen höheren Anteil an Polyamidfasern und einen geringeren Anteil an Viskosefasern im Vergleich zu der eine Innenseite der Hülle bildenden Schicht aufweist. Entsprechend weist die eine Innenseite der Hülle bildende Schicht zur Erhöhung der Kapillarwirkung und somit der Exsudataufnahme einen geringeren Anteil an Polyamidfasern, dafür aber einen höheren Anteil an Viskosefasern im Vergleich zu der eine Außenseite der Hülle bildenden Schicht auf. Dabei kann der mittlere Anteil an Polyamid und Viskose wie zuvor angegeben sein. Das heißt, die beiden Schichten zusammen betrachtet können aus 50 Gew.-% bis 60 Gew.-%, insbesondere etwa 55 Gew.-%, Polyamidfasern und 40 Gew.-% bis 50 Gew.-%, insbesondere etwa 45 Gew.-%, Viskosefasern bezogen auf das Gesamtgewicht der zweiten Vliesstoffschicht bestehen. Mit dem in dieser besonders vorteilhaften Ausführungsform beschriebenen zweischichtigen Aufbau kann eine im hohen Maße atmungsaktive, flüssigkeitsdurchlässige und nichtverklebende Wundkontaktschicht für die Wundauflage bereitgestellt werden.

In der Regel sind die erste und die zweite Vliesstoffschicht der Hülle des Wundkissens in ihren Randbereichen derart miteinander verbunden, dass die Hülle eine Tasche ausbildet, in welcher der absorbierende Kern enthalten ist. Vorzugsweise sind die erste und die zweite Vliesstoffschicht der Hülle des Wundkissens durch Ultraschallverschweißen miteinander verbunden. Als alternative, vorliegend aber weniger bevorzugte Verbindungstechniken sind Verkleben oder Vernähen denkbar.

Die in dem absorbierenden Kern des Wundkissens enthaltenen SAP-Partikel bestehen bevorzugt aus einem Polyacrylat. Polyacrylate können sehr hohe Flüssigkeitsmengen binden und sind daher hervorragend für die vorliegende Erfindung als absorbierendes Mittel geeignet.

Üblicherweise umfasst der absorbierende Kern des Wundkissens weiterhin ein absorbierendes Fasermaterial, insbesondere hydrophile Zellulosefasern. Das Fasermaterial ist typischerweise flockenartig ausgestaltet, weshalb es regelmäßig auch als "Fluff" bezeichnet wird. In dem Fasermaterial sind die SAP-Partikel normalerweise gleichmäßig verteilt eingestreut. Somit kann ein sehr wirkungsvoller, hoch-absorptionsfähiger Kern für das Wundkissen erhalten werden. Der absorbierende Kern des Wundkissens kann zum Beispiel 30 Gew.-% bis 60 Gew.-% SAP-Partikel und 40 Gew.-% bis 70 Gew.-% absorbierendes Fasermaterial umfassen. Bevorzugt umfasst der absorbierende Kern 40 Gew.-% bis 50 Gew.-% SAP-Partikel und 50 Gew.-% bis 60 Gew.-% absorbierendes Fasermaterial. Die Angaben beziehen sich dabei jeweils auf das Gesamtgewicht des absorbierenden Kerns.

Besonders vorteilhaft ist es, wenn der absorbierende Kern des Wundkissens zusätzlich von einer Verteilerschicht, insbesondere einer Verteilerschicht bestehend aus einem hydrophilen Zellulosegewebe oder Zellulosevlies, eingefasst ist. Die Verteilerschicht ist zwischen der Hülle des Wundkissens und dem absorbierenden Kern des Wundkissens angeordnet und funktionsbedingt zwingend flüssigkeitsdurchlässig und wasserdampfdurchlässig ausgebildet. Die Verteilerschicht hat nämlich die Aufgabe, das Wundexsudat im Wundkissen zu verteilen und damit dessen Absorptionsvermögen zu verbessern. Die Verteilerschicht kann in der Art einer geschlossenen Hülle ausgebildet sein, die den absorbierenden Kern des Wundkissens enthält. In dieser Ausführungsform der Erfindung weist das Wundkissen dann folglich zwei Hülllagen auf. Eine erste, äußere Hülle mit Wundkontakt und eine zweite, innere Hülle, welche die Verteilerschicht darstellt und ihrerseits von der ersten, äußeren Hülle umschlossen ist. In einer einfacheren, aber ebenso gut funktionierenden Version kann die Verteilerschicht eine offene Umhüllung für den absorbierenden Kern ausbilden. Zum Beispiel kann die Verteilerschicht den absorbierenden Kern dabei schlauchartig einfassen, wobei dann zwei Enden der Umhüllung offen bleiben. Hierfür kann ein Streifen der Verteilerschicht auf sich selbst zurückgelegt werden. Der dabei gebildete Zwischenraum nimmt dann den absorbierenden Kern auf.

Das Wundkissen, insbesondere der absorbierende Kern des Wundkissens, kann eine Saugleistung von mindestens 50g/100cm², vorzugsweise von mindestens 100g/100cm² und insbesondere von mindestens 140g/100cm², gemessen nach DIN EN 13726-1:2002-06 (Kapitel 3.2), aufweisen. Diese hohen Saugleistungen können durch eine geeignete Menge an SAP-Partikeln im absorbierenden Kern des Wundkissens problemlos erzielt werden. Die Saugleistung des Wundkissens, insbesondere des absorbierenden Kerns des Wundkissens, kann maximal 220g/100cm², 250g/100cm² oder 300g/100cm², gemessen nach DIN EN 13726-1:2002-06 (Kapitel 3.2), betragen. Bevorzugt weist das Wundkissen, insbesondere der absorbierende Kern des Wundkissens, eine Saugleistung von 140g/100cm² bis 220g/100cm², gemessen nach DIN EN 13726-1:2002-06 (Kapitel 3.2), auf.

Im Rahmen der vorliegenden Erfindung besteht das Wundkissen üblicherweise aus der Hülle, optional der Verteilerschicht und dem absorbierenden Kern, wobei der absorbierende Kern vorzugsweise aus dem absorbierenden Fasermaterial und den SAP-Partikeln besteht. Das Wundkissen kann dann eine Vielzahl von Vorteilen aufweisen. So kann das Wundkissen dann flexibel, weich, hoch-absorbierend, atmungsaktiv, atraumatisch sowie kostengünstig sein.

Gegenstand der vorliegenden Erfindung ist wie bereits erwähnt auch ein Verfahren zur Herstellung der erfindungsgemäßen Wundauflage. Dieses Verfahren umfasst die nachfolgenden Schritte:
i. Bereitstellen einer Rückschicht bestehend aus einem Vliesstoff,
ii. Bereitstellen einer ersten adhäsiven Schicht,
iii. Bereitstellen eines Wundkissens umfassend eine Hülle und einen absorbierenden Kern mit SAP-Partikeln, wobei das Wundkissen kleiner ausgebildet ist als die Rückschicht und die erste adhäsive Schicht,
iv. optional Bereitstellen einer einteiligen oder mehrteiligen, insbesondere zweiteiligen, Abziehfolie,
v. Verbinden der Rückschicht, der ersten adhäsiven Schicht und des Wundkissens miteinander, derart, dass die erste adhäsive Schicht zwischen der Rückschicht und dem Wundkissen angeordnet ist und dass die Rückschicht und die erste adhäsive Schicht das Wundkissen überfangen, so dass die Wundauflage einen adhäsiven Rand aufweist,
vi. optional Bedecken des adhäsiven Randes und einer wundzugewandten Seite der Hülle des Wundkissens mit der Abziehfolie.

Allerdings umfasst das Verfahren erfindungsgemäß keinen Schritt, in dem ein Material beziehungsweise eine Schicht mit dem adhäsiven Rand und einer wundzugewandten Seite der Hülle des Wundkissen unlösbar verbunden wird.

Das Herstellungsverfahren bezieht sich auch auf die voranstehend beschriebenen speziellen Ausführungsformen der Wundauflage. Das heißt, die weiteren Merkmale der voranstehend beschriebenen speziellen Ausführungsformen der Wundauflage sind auch Bestandteil des Herstellungsverfahrens.

Entsprechend kann das Herstellungsverfahren in einer besonders bevorzugten Variante die nachfolgenden Schritte umfassen:
i. Bereitstellen einer Rückschicht bestehend aus einem Vliesstoff,
ii. Bereitstellen einer ersten adhäsiven Schicht, indem eine wundzugewandte Seite der Rückschicht mit einem Adhäsiv beschichtet wird,
iii. Bereitstellen eines Wundkissens umfassend eine Hülle und einen absorbierenden Kern mit SAP-Partikeln, wobei das Wundkissen kleiner ausgebildet ist als die Rückschicht und die erste adhäsive Schicht,
iv. optional Bereitstellen einer einteiligen oder mehrteiligen, insbesondere zweiteiligen, Abziehfolie,
v. Verbinden der Rückschicht und des Wundkissens miteinander, indem die erste adhäsive Schicht mit einer wundabgewandten Seite der Hülle des Wundkissens in Kontakt gebracht wird, wobei die Rückschicht und die erste adhäsive Schicht das Wundkissen überfangen und einen adhäsiven Rand ausbilden,
vi. optional Bedecken des adhäsiven Randes und einer wundzugewandten Seite der Hülle des Wundkissens mit der Abziehfolie.

Die bevorzugte Variante des Verfahrens folgt gleichfalls der Maßgabe, dass es keinen Schritt umfasst, in dem ein Material beziehungsweise eine Schicht mit dem adhäsiven Rand und einer wundzugewandten Seite der Hülle des Wundkissen unlösbar verbunden wird.

### Figuren

Mit den nachfolgend beschriebenen Figuren soll die Erfindung exemplarisch näher erläutert und veranschaulicht werden. Dabei können in den Figuren ähnliche Strukturelemente mit denselben Bezugszeichen ausgewiesen sein.

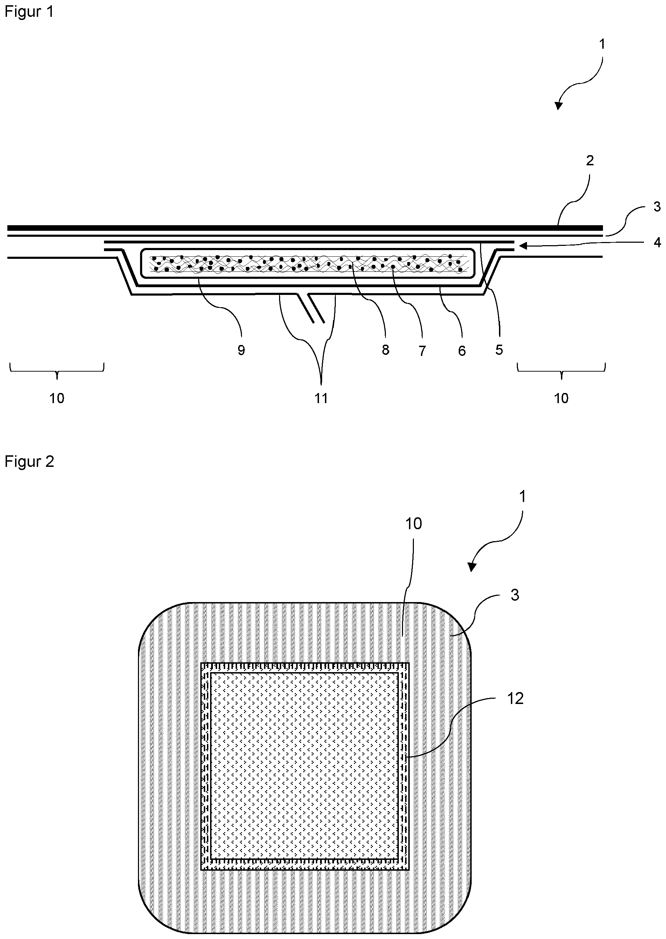

**Figur 1** zeigt eine Schnittansicht einer erfindungsgemäßen Wundauflage **1** in einer ersten Ausführungsform. Die Wundauflage umfasst eine Rückschicht **2,** welche aus einem Vliesstoff besteht. Die Rückschicht kann perforiert (nicht dargestellt) und aus Polyesterfasern gebildet sein. Eine wundzugewandte Seite der Rückschicht **2** ist mit einem Adhäsiv **3,** beispielsweise einem synthetischen Kautschuk-Kleber, beschichtet. Die erste adhäsive Schicht **3** bildet vorteilhafterweise ein gestreiftes Muster aus, welches auf der gesamten wundzugewandten Seite der Rückschicht **2** vorhanden ist. Weiterhin umfasst die Wundauflage **1** ein Wundkissen **4.** Das Wundkissen **4** umfasst eine Hülle, welche vorteilhafterweise aus zwei in ihren Randbereichen miteinander verbundenen Vliesstoffschichten **5, 6** gebildet wird. Die wundabgewandte Vliesstoffschicht **5** der Hülle ist vorzugsweise hydrophob sowie im Wesentlichen flüssigkeitsundurchlässig ausgebildet und kann zum Beispiel aus Polypropylenfasern bestehen. Die wundzugewandte Vliesstoffschicht **6** der Hülle bildet eine flüssigkeitsdurchlässige Wundkontaktschicht der Wundauflage **1** und ist vorzugsweise hydrophil ausgebildet. Wie bereits beschrieben kann die Vliesstoffschicht **6** der Hülle zweischichtig aufgebaut sein (nicht separat in **Figur 1** gezeigt) und aus Polyamidfasern in Mischung mit Viskosefasern bestehen. Weiterhin umfasst das Wundkissen **4** einen absorbierenden Kern mit SAP-Partikeln **7.** Im dargestellten Fall besteht der absorbierende Kern aus den SAP-Partikeln **7** und einem absorbierenden Fasermaterial **8.** Bei dem absorbierenden Fasermaterial **8** kann es sich insbesondere um hydrophile Zellulosefasern handeln. Schließlich umfasst das Wundkissen **4** bevorzugt noch eine Verteilerschicht **9.** Diese kann aus einem dünnen, hydrophilen Zellulosevlies bestehen und umgibt den absorbierenden Kern für eine verbesserte Flüssigkeitsverteilung innerhalb des Wundkissens **4.** Wie aus **Figur 1** hervorgeht, überfängt die adhäsiv beschichtete Rückschicht **2, 3** das Wundkissen **4,** so dass ein adhäsiver Rand **10** der Wundauflage **1** ausgebildet werden kann. Mit dem adhäsiven Rand **10** wird die Wundauflage **1** am Körper des Patienten befestigt, wobei der adhäsive Rand **10** üblicherweise nur an intakter Haut befestigt wird. In **Figur 1** ist auch noch eine zweiteilige Abziehfolie **11** mit an sich aus dem Stand der Technik bekannter Ausgestaltung angedeutet.
**Figur 2** zeigt die Wundauflage **1** aus **Figur 1** mit Blick auf die Unterseite ohne die Abziehfolie **11.** Deutlich zu erkennen ist insbesondere der für Inselverbände typische überstehende Kleberand **10** der adhäsiven Rückschicht **2, 3,** welcher die Wundauflage **4** vollumfänglich umgibt. Darüber hinaus sind die gestreifte Klebstoffbeschichtung **3** sowie die mit Ultraschall erzeugten Schweißnähte **12** zur Verbindung der beiden Hülllagen **5, 6** in **Figur 2** angedeutet.

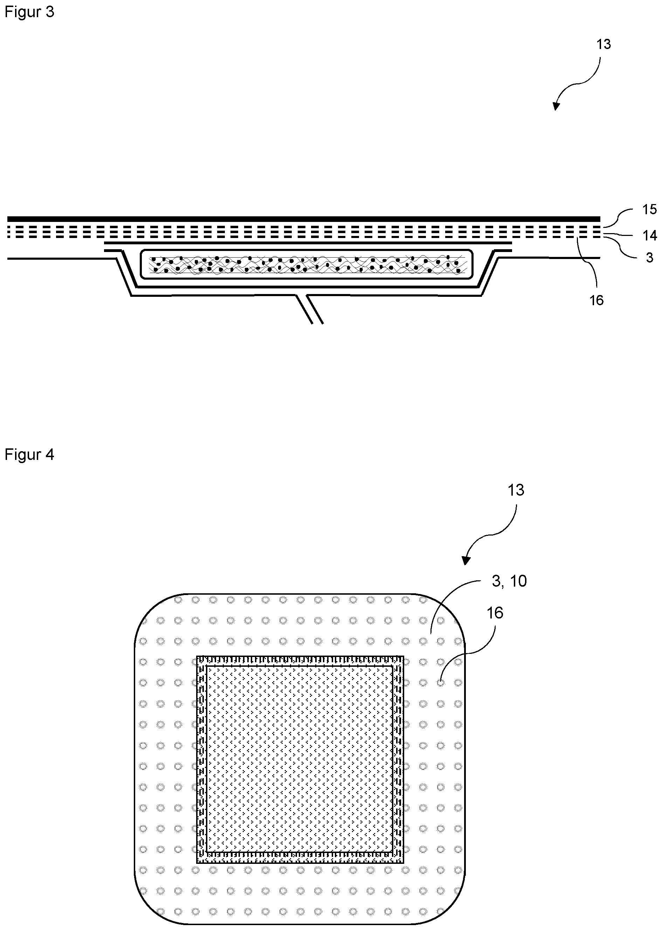

**Figur 3** zeigt eine Schnittansicht einer erfindungsgemäßen Wundauflage **13** in einer zweiten Ausführungsform. Die Wundauflage **13** unterscheidet sich von der Wundauflage **1** dadurch, dass die adhäsive Beschichtung **3** nicht unmittelbar mit der Rückschicht **2** verbunden ist, sondern die Unterseite einer zusätzlich vorhandenen, perforierten Folie **14** bedeckt. Vorzugsweise wird für die Beschichtung **3** in dieser Ausführungsform ein schonender Silikonkleber eingesetzt. Die Oberseite beziehungsweise die wundabgewandte Seite der **Folie 14** ist gleichfalls adhäsiv beschichtet, vorzugsweise mit einem Acrylatkleber. Diese weitere, zweite adhäsive Schicht **15** stellt dann die Verbindung der Folie **14** mit der Rückschicht **2** her. Das Wundkissen **4** ist wie bei der Wundauflage **1** über die erste adhäsive Schicht **3** mit den die Rückseite der Wundauflage bildenden Lagen dauerhaft verbunden. Bei der Wundauflage **13** ist also eine beidseitig adhäsiv beschichtete Folie zwischen der Rückschicht **2** und dem Wundkissen **4** vorhanden, welche aufgrund der durchgehenden Perforationen **16** noch vergleichsweise atmungsaktiv sein kann. Die Perforationen **16** sind bevorzugt auf der gesamten Fläche der adhäsiven Folie **3, 14, 15** vorhanden. Veranschaulicht ist dies in **Figur 4****,** welche die Wundauflage **13** mit Blick auf die Unterseite ohne Abziehfolie **11** zeigt.

Die in den Figuren beispielhaft gezeigten Wundauflagen sind im Wesentlichen rechteckig ausgestaltet. Sie können aber auch andere Formen haben und zum Beispiel rund oder oval ausgestaltet sein oder eine für die sakrale Anwendung angepasste Form aufweisen. Typischerweise werden die Wundauflagen in verschiedenen Größen und Formaten angeboten. Zum Beispiel könnte bei der Wundauflage **1** das Wundkissen **4** eine Abmessung von 10 cm x 10 cm und die adhäsive Rückschicht **2, 3** eine Abmessung von 15 cm x 15 cm aufweisen, so dass ein adhäsiver Rand **10** mit einer Breite von 2,5 cm ausgebildet werden kann.

## Patentansprüche

1. Wundauflage (1, 13) umfassend
- eine Rückschicht (2) aus einem Vliesstoff,
- ein Wundkissen (4) umfassend eine Hülle (5, 6) und einen absorbierenden Kern mit SAP-Partikeln (7),
- eine erste adhäsive Schicht (3), welche zwischen der Rückschicht (2) und dem Wundkissen (4) angeordnet ist,
wobei die Rückschicht (2) und die erste adhäsive Schicht (3) das Wundkissen (4) überfangen, so dass die Wundauflage einen adhäsiven Rand (10) aufweist, und wobei die Wundauflage keine mit dem adhäsiven Rand (10) und einer wundzugewandten Seite (6) der Hülle des Wundkissens unlösbar verbundene Schicht umfasst, so dass die Wundauflage mit dem adhäsiven Rand (10) an einer Wundstelle befestigt werden kann und die wundzugewandte Seite (6) der Hülle des Wundkissens eine Wundkontaktschicht der Wundauflage bilden kann.

2. Wundauflage nach Anspruch 1, wobei die Rückschicht (2) perforiert ist, wobei vorzugsweise eine Vielzahl von Perforationen im Wesentlichen gleichmäßig über die Rückschicht (2) verteilt vorhanden sind.

3. Wundauflage nach Anspruch 1 oder 2, wobei die Rückschicht (2), vorzugsweise die Rückschicht (2) in Verbindung mit der ersten adhäsiven Schicht (3) und insbesondere die Wundauflage, eine Wasserdampfdurchlässigkeit von mindestens 3000 g/m²/24h, vorzugsweise von mindestens 3500 g/m²/24h und insbesondere von mindestens 4000 g/m²/24h, gemessen nach DIN EN 13726-2:2002-06, aufweist.

4. Wundauflage nach einem der vorangehenden Ansprüche, wobei die erste adhäsive Schicht (3) als Beschichtung einer wundzugewandten Seite der Rückschicht (2) ausgebildet ist.

5. Wundauflage nach einem der vorangehenden Ansprüche, wobei die erste adhäsive Schicht (3) perforiert ist oder voneinander beabstandete Streifen ausbildet, wobei die erste adhäsive Schicht (3) vorzugsweise ein gelochtes oder gestreiftes Muster ausbildet, welches auf der gesamten wundzugewandten Seite der Rückschicht (2) vorhanden ist.

6. Wundauflage nach einem der vorangehenden Ansprüche, wobei die erste adhäsive Schicht (3) einen Acrylatkleber, einen synthetischen Kautschuk-Kleber oder einen Silikonkleber umfasst.

7. Wundauflage nach einem der vorangehenden Ansprüche, wobei die Wundauflage aus der Rückschicht (2), dem Wundkissen (4), der ersten adhäsiven Schicht (3) und optional einer einteiligen oder mehrteiligen, insbesondere zweiteiligen, Abziehfolie (11) besteht, wobei die Abziehfolie (11) den adhäsiven Rand (10) und die wundzugewandte Seite (6) der Hülle des Wundkissens bedeckt und von der Wundauflage abgezogen werden kann.

8. Wundauflage nach einem der Ansprüche 1 bis 3, wobei zwischen der Rückschicht (2) und dem Wundkissen (4) eine perforierte Folie (14), insbesondere eine perforierte Polyurethanfolie, angeordnet ist, welche das Wundkissen (4) überfängt, wobei eine wundabgewandte Seite der Folie (14) mit einem Acrylatkleber beschichtet ist und eine wundzugewandte Seite der Folie (14) mit einem Silikonkleber beschichtet ist, wobei der Acrylatkleber eine zweite adhäsive Schicht (15) der Wundauflage bildet und zur Verbindung der Folie (14) mit der Rückschicht (2) dient und wobei der Silikonkleber die erste adhäsive Schicht (3) der Wundauflage bildet, und zur Verbindung der Folie (14) mit dem Wundkissen (4) und zur Befestigung der Wundauflage an der Wundstelle dient.

9. Wundauflage nach Anspruch 8, wobei die Wundauflage aus der Rückschicht (2), dem Wundkissen (4), der beidseitig adhäsiv beschichteten Folie (3, 14, 15) nach Anspruch 8 und optional einer einteiligen oder mehrteiligen, insbesondere zweiteiligen, Abziehfolie (11) besteht, wobei die Abziehfolie (11) den adhäsiven Rand (10) und die wundzugewandte Seite (6) der Hülle des Wundkissens bedeckt und von der Wundauflage abgezogen werden kann.

10. Wundauflage nach einem der vorangehenden Ansprüche, wobei die Hülle des Wundkissens (4) aus einer ersten, wundabgewandten hydrophoben Vliesstoffschicht (5) und einer zweiten, wundzugewandten hydrophilen Vliesstoffschicht (6) besteht, wobei die zweite, wundzugewandte hydrophile Vliesstoffschicht (6) die Wundkontaktschicht der Wundauflage bildet.

11. Wundauflage nach einem der vorangehenden Ansprüche, wobei der absorbierende Kern des Wundkissens (4) weiterhin ein absorbierendes Fasermaterial (8), insbesondere hydrophile Zellulosefasern, umfasst.

12. Wundauflage nach einem der vorangehenden Ansprüche, wobei der absorbierende Kern (7, 8) des Wundkissens (4) zusätzlich von einer Verteilerschicht (9), insbesondere einer Verteilerschicht (9) aus einem hydrophilen Zellulosegewebe oder Zellulosevlies, eingefasst ist, wobei die Verteilerschicht (9) zwischen der Hülle (5, 6) des Wundkissens und dem absorbierenden Kern (7, 8) des Wundkissens angeordnet ist.

13. Wundauflage nach einem der vorangehenden Ansprüche, wobei das Wundkissen (4), insbesondere der absorbierende Kern (7, 8) des Wundkissens, eine Saugleistung von mindestens 50g/100cm², vorzugsweise von mindestens 100g/100cm² und insbesondere von mindestens 140g/100cm², gemessen nach DIN EN 13726-1:2002-06 (Kapitel 3.2), aufweist.

14. Wundauflage nach einem der vorangehenden Ansprüche, wobei das Wundkissen (4) aus der Hülle (5, 6), optional der Verteilerschicht (9) und dem absorbierenden Kern (7, 8) besteht, wobei der absorbierende Kern vorzugsweise aus dem absorbierenden Fasermaterial (8) und den SAP-Partikeln (7) besteht.

15. Verfahren zur Herstellung einer Wundauflage (1, 13) nach einem der vorangehenden Ansprüche, umfassend die Schritte:
i. Bereitstellen einer Rückschicht (2) aus einem Vliesstoff,
ii. Bereitstellen einer ersten adhäsiven Schicht (3),
iii. Bereitstellen eines Wundkissens (4) umfassend eine Hülle (5, 6) und einen absorbierenden Kern mit SAP-Partikeln (7), wobei das Wundkissen (4) kleiner ausgebildet ist als die Rückschicht (2) und die erste adhäsive Schicht (3),
iv. optional Bereitstellen einer einteiligen oder mehrteiligen, insbesondere zweiteiligen, Abziehfolie (11),
v. Verbinden der Rückschicht (2), der ersten adhäsiven Schicht (3) und des Wundkissens (4) miteinander, derart, dass die erste adhäsive Schicht (3) zwischen der Rückschicht (2) und dem Wundkissen (4) angeordnet ist und dass die Rückschicht (2) und die erste adhäsive Schicht (3) das Wundkissen (4) überfangen, so dass die Wundauflage einen adhäsiven Rand (10) aufweist,
vi. optional Bedecken des adhäsiven Randes (10) und einer wundzugewandten Seite (6) der Hülle des Wundkissens mit der Abziehfolie (11),
wobei das Verfahren keinen Schritt umfasst, in dem ein Material mit dem adhäsiven Rand (10) und einer wundzugewandten Seite (6) der Hülle des Wundkissen unlösbar verbunden wird.

## Claims

1. Wound dressing (1, 13) comprising
- a backing layer (2) composed of a nonwoven,
- a wound pad (4) comprising a shell (5, 6) and an absorbent core containing SAP particles (7),
- a first adhesive layer (3) arranged between the backing layer (2) and the wound pad (4),
wherein the backing layer (2) and the first adhesive layer (3) cover the wound pad (4), such that the wound dressing has an adhesive edge (10), and wherein the wound dressing does not comprise a layer which is non-releasably connected to the adhesive edge (10) and to a wound-facing side (6) of the shell of the wound pad, such that the wound dressing can be fastened at a wound site by means of the adhesive edge (10) and the wound-facing side (6) of the shell of the wound pad can form a wound contact layer of the wound dressing.

2. Wound dressing according to Claim 1, wherein the backing layer (2) is perforated, wherein a multiplicity of perforations are preferably distributed substantially uniformly over the backing layer (2).

3. Wound dressing according to Claim 1 or 2, wherein the backing layer (2), preferably the backing layer (2) in connection with the first adhesive layer (3) and in particular the wound dressing, has a moisture vapour transmission rate of at least 3000 g/m²/24 h, preferably of at least 3500 g/m²/24 h and in particular of at least 4000 g/m²/24 h, measured according to DIN EN 13726-2:2002-06.

4. Wound dressing according to one of the preceding claims, wherein the first adhesive layer (3) is in the form of a coating of a wound-facing side of the backing layer (2).

5. Wound dressing according to one of the preceding claims, wherein the first adhesive layer (3) is perforated or forms strips which are spaced apart from one another, wherein the first adhesive layer (3) preferably forms a perforated or striped pattern which is present on the entire wound-facing side of the backing layer (2).

6. Wound dressing according to one of the preceding claims, wherein the first adhesive layer (3) comprises an acrylate adhesive, a synthetic rubber adhesive or a silicone adhesive.

7. Wound dressing according to one of the preceding claims, wherein the wound dressing consists of the backing layer (2), the wound pad (4), the first adhesive layer (3) and optionally a one-part or multi-part, in particular two-part, peel-off film (11), wherein the peel-off film (11) covers the adhesive edge (10) and the wound-facing side (6) of the shell of the wound pad and can be peeled off from the wound dressing.

8. Wound dressing according to one of Claims 1 to 3, wherein a perforated film (14), in particular a perforated polyurethane film, is arranged between the backing layer (2) and the wound pad (4) and covers the wound pad (4), wherein a side of the film (14) facing away from the wound is coated with an acrylate adhesive and a wound-facing side of the film (14) is coated with a silicone adhesive, wherein the acrylate adhesive forms a second adhesive layer (15) of the wound dressing and is used to connect the film (14) to the backing layer (2) and wherein the silicone adhesive forms the first adhesive layer (3) of the wound dressing and is used to connect the film (14) to the wound pad (4) and to fasten the wound dressing at the wound site.

9. Wound dressing according to Claim 8, wherein the wound dressing consists of the backing layer (2), the wound pad (4), the film (3, 14, 15) according to Claim 8 which is adhesively coated on both sides and optionally a one-part or multi-part, in particular two-part, peel-off film (11), wherein the peel-off film (11) covers the adhesive edge (10) and the wound-facing side (6) of the shell of the wound pad and can be peeled off from the wound dressing.

10. Wound dressing according to one of the preceding claims, wherein the shell of the wound pad (4) consists of a first hydrophobic nonwoven layer (5) which faces away from the wound and a second, wound-facing hydrophilic nonwoven layer (6), wherein the second, wound-facing hydrophilic nonwoven layer (6) forms the wound contact layer of the wound dressing.

11. Wound dressing according to one of the preceding claims, wherein the absorbent core of the wound pad (4) further comprises an absorbent fibre material (8), in particular hydrophilic cellulose fibres.

12. Wound dressing according to one of the preceding claims, wherein the absorbent core (7, 8) of the wound pad (4) is additionally enclosed by a distributor layer (9), in particular a distributor layer (9) composed of a hydrophilic woven cellulose fabric or cellulose nonwoven, wherein the distributor layer (9) is arranged between the shell (5, 6) of the wound pad and the absorbent core (7, 8) of the wound pad.

13. Wound dressing according to one of the preceding claims, wherein the wound pad (4), in particular the absorbent core (7, 8) of the wound pad, has an absorbency of at least 50 g/100 cm², preferably of at least 100 g/100 cm² and in particular of at least 140 g/100 cm², measured according to DIN EN 13726-1:2002-06 (section 3.2) .

14. Wound dressing according to one of the preceding claims, wherein the wound pad (4) consists of the shell (5, 6), optionally the distributor layer (9), and the absorbent core (7, 8), wherein the absorbent core preferably consists of the absorbent fibre material (8) and the SAP particles (7).

15. Method for producing a wound dressing (1, 13) according to one of the preceding claims, comprising the steps:
i. providing a backing layer (2) composed of a nonwoven,
ii. providing a first adhesive layer (3),
iii. providing a wound pad (4) comprising a shell (5, 6) and an absorbent core containing SAP particles (7), wherein the wound pad (4) is smaller than the backing layer (2) and the first adhesive layer (3),
iv. optionally providing a one-part or multi-part, in particular two-part, peel-off film (11),
v. connecting the backing layer (2), the first adhesive layer (3) and the wound pad (4) to one another, in such a way that the first adhesive layer (3) is arranged between the backing layer (2) and the wound pad (4) and the backing layer (2) and the first adhesive layer (3) cover the wound pad (4), such that the wound dressing has an adhesive edge (10),
vi. optionally covering the adhesive edge (10) and a wound-facing side (6) of the shell of the wound pad with the peel-off film (11),
wherein the method does not comprise a step in which a material is non-releasably connected to the adhesive edge (10) and to a wound-facing side (6) of the shell of the wound pad.

## Revendications

1. Pansement (1, 13) comprenant
- une couche arrière (2) en un tissu non tissé,
- un coussinet pour plaie (4) comprenant une enveloppe (5, 6) et un noyau absorbant comportant des particules de SAP (7),
- une première couche adhésive (3) qui est agencée entre la couche arrière (2) et le coussinet pour plaie (4),
la couche arrière (2) et la première couche adhésive (3) couvrant le coussinet pour plaie (4), de telle sorte que le pansement présente un bord adhésif (10), et
le pansement ne comprenant pas de couche reliée de manière inamovible au bord adhésif (10) et à un côté (6) de l'enveloppe du coussinet pour plaie tourné vers la plaie, de telle sorte que le pansement peut être fixé à un site de plaie avec le bord adhésif (10) et le côté (6) de l'enveloppe du coussinet pour plaie tourné vers la plaie peut former une couche de contact avec la plaie du pansement.

2. Pansement selon la revendication 1, dans lequel la couche arrière (2) est perforée, une pluralité de perforations étant de préférence réparties de manière essentiellement uniforme sur la couche arrière (2).

3. Pansement selon la revendication 1 ou 2, dans lequel la couche arrière (2), de préférence la couche arrière (2) en combinaison avec la première couche adhésive (3) et notamment le pansement, présente une perméabilité à la vapeur d'eau d'au moins 3 000 g/m²/24 h, de préférence d'au moins 3 500 g/m²/24 h et notamment d'au moins 4 000 g/m²/24 h, mesurée selon la norme DIN EN 13726-2:2002-06.

4. Pansement selon l'une quelconque des revendications précédentes, dans lequel la première couche adhésive (3) est réalisée sous forme de revêtement d'un côté de la couche arrière (2) tourné vers la plaie.

5. Pansement selon l'une quelconque des revendications précédentes, dans lequel la première couche adhésive (3) est perforée ou réalise des bandes espacées les unes des autres, la première couche adhésive (3) réalisant de préférence un motif perforé ou rayé qui est présent sur tout le côté de la couche arrière (2) tourné vers la plaie.

6. Pansement selon l'une quelconque des revendications précédentes, dans lequel la première couche adhésive (3) comprend un adhésif d'acrylate, un adhésif en caoutchouc synthétique ou un adhésif de silicone.

7. Pansement selon l'une quelconque des revendications précédentes, dans lequel le pansement est constitué de la couche arrière (2), du coussinet pour plaie (4), de la première couche adhésive (3) et éventuellement d'une feuille détachable (11) en une ou plusieurs parties, notamment en deux parties, la feuille détachable (11) recouvrant le bord adhésif (10) et le côté (6) de l'enveloppe du coussinet pour plaie tourné vers la plaie et pouvant être détachée du pansement.

8. Pansement selon l'une quelconque des revendications 1 à 3, dans lequel une feuille perforée (14), notamment une feuille de polyuréthane perforée, est agencée entre la couche arrière (2) et le coussinet pour plaie (4), laquelle feuille couvre le coussinet pour plaie (4), un côté de la feuille (14) détourné de la plaie étant revêtu d'un adhésif acrylate et un côté de la feuille (14) tourné vers la plaie étant revêtu d'un adhésif silicone, l'adhésif d'acrylate formant une deuxième couche adhésive (15) du pansement et servant à relier la feuille (14) à la couche arrière (2) et l'adhésif de silicone formant la première couche adhésive (3) du pansement et servant à relier la feuille (14) au coussinet pour plaie (4) et à fixer le pansement au site de plaie.

9. Pansement selon la revendication 8, dans lequel le pansement est constitué de la couche arrière (2), du coussinet pour plaie (4), de la feuille (3, 14, 15) revêtue d'adhésif sur les deux côtés selon la revendication 8 et éventuellement d'une feuille détachable (11) en une ou plusieurs parties, notamment en deux parties, la feuille détachable (11) recouvrant le bord adhésif (10) et le côté (6) de l'enveloppe du coussinet pour plaie tourné vers la plaie et pouvant être retirée du pansement.

10. Pansement selon l'une quelconque des revendications précédentes, dans lequel l'enveloppe du coussinet pour plaie (4) est constituée d'une première couche de tissu non tissé hydrophobe (5) détournée de la plaie et d'une deuxième couche de tissu non tissé hydrophile (6) tournée vers la plaie, la deuxième couche de tissu non tissé hydrophile (6) tournée vers la plaie formant la couche de contact avec la plaie du pansement.

11. Pansement selon l'une quelconque des revendications précédentes, dans lequel le noyau absorbant du coussinet pour plaie (4) comprend en outre un matériau fibreux absorbant (8), notamment des fibres de cellulose hydrophiles.

12. Pansement selon l'une quelconque des revendications précédentes, dans lequel le noyau absorbant (7, 8) du coussinet pour plaie (4) est en outre entouré d'une couche de répartition (9), notamment d'une couche de répartition (9) composée d'un tissu de cellulose hydrophile ou d'un non-tissé de cellulose, la couche de répartition (9) étant agencée entre l'enveloppe (5, 6) du coussinet pour plaie et le noyau absorbant (7, 8) du coussinet pour plaie.

13. Pansement selon l'une quelconque des revendications précédentes, dans lequel le coussinet pour plaie (4), notamment le noyau absorbant (7, 8) du coussinet pour plaie, présente une capacité d'absorption d'au moins 50 g/100 cm², de préférence d'au moins 100 g/100 cm² et notamment d'au moins 140 g/100 cm², mesurée selon la norme DIN EN 13726-1:2002-06 (chapitre 3.2).

14. Pansement selon l'une quelconque des revendications précédentes, dans lequel le coussinet pour plaie (4) est constitué de l'enveloppe (5, 6), éventuellement de la couche de répartition (9) et du noyau absorbant (7, 8), le noyau absorbant étant de préférence constitué du matériau fibreux absorbant (8) et des particules de SAP (7) .

15. Procédé de fabrication d'un pansement (1, 13) selon l'une quelconque des revendications précédentes, comprenant les étapes suivantes :
i. la fourniture d'une couche arrière (2) en un tissu non tissé,
ii. la fourniture d'une première couche adhésive (3),
iii. la fourniture d'un coussinet pour plaie (4) comprenant une enveloppe (5, 6) et un noyau absorbant avec des particules de SAP (7), le coussinet pour plaie (4) étant réalisé plus petit que la couche arrière (2) et la première couche adhésive (3),
iv. éventuellement la fourniture d'une feuille détachable (11) en une ou plusieurs parties, notamment en deux parties,
v. la liaison de la couche arrière (2), de la première couche adhésive (3) et du coussinet pour plaie (4) les uns aux autres, de telle sorte que la première couche adhésive (3) est agencée entre la couche arrière (2) et le coussinet pour plaie (4) et que la couche arrière (2) et la première couche adhésive (3) couvrent le coussinet pour plaie (4), de telle sorte que le pansement présente un bord adhésif (10),
vi. éventuellement le recouvrement du bord adhésif (10) et d'un côté (6) de l'enveloppe du coussinet pour plaie tourné vers la plaie avec la feuille détachable (11),
le procédé ne comprenant pas d'étape dans laquelle un matériau est relié de manière inamovible au bord adhésif (10) et à un côté (6) de l'enveloppe du coussinet pour plaie tourné vers la plaie.
